# EUROPÄISCHE PATENTSCHRIFT

(11) **EP 4 401 668 B1**
(45) Veröffentlichungstag und Bekanntmachung des Hinweises auf die Patenterteilung: **02.09.2026**
(21) Anmeldenummer: 22792789.4
(22) Anmeldetag: 16.09.2022
(51) Int. Cl.: A61B 90/00, A61B 90/30, A61B 17/00

(54) **BELEUCHTUNGSVORRICHTUNG ZUM BELEUCHTEN EINER OPERATIONSWUNDE**
ILLUMINATING DEVICE FOR ILLUMINATING A SURGICAL WOUND
DISPOSITIF D'ÉCLAIRAGE POUR ÉCLAIRER UNE PLAIE CHIRURGICALE

(30) Priorität: 17.09.2021 DE 102021124083
(43) Veröffentlichungstag der Anmeldung: 24.07.2024
(73) Patentinhaber: Dr. Mach GmbH & Co. KG, 85567 Grafing (DE)
(72) Erfinder: ZACHMANN, Gabriel, 37075 Göttingen (DE); MÜHLENBROCK, Andre Richard, 28209 Bremen (DE); KOHRS, Peter, 38527 Meine (DE); FOX, Adrian Urs, 83561 Ramerberg (DE)
(74) Vertreter: Weickmann & Weickmann PartmbB
(86) Internationale Anmeldenummer: PCT/EP2022/075824
(87) Internationale Veröffentlichungsnummer: WO 2023/041732

(56) Entgegenhaltungen:
- EP-A1- 2 434 202
- EP-A1- 2 495 487
- DE-A1- 102013 012 231

## Beschreibung

Die vorliegende Erfindung betrifft eine Beleuchtungsvorrichtung zum Beleuchten einer Operationswunde, welche an der Oberfläche des Körpers eines Patienten eine Wundenöffnungsfläche aufweist und sich in einer Tiefenerstreckungsrichtung in den Körper des Patienten hinein erstreckt.

In heutigen Anwendungen wird die ideale Ausleuchtung einer Operationswunde, auch "Situs" genannt, dadurch erreicht, dass der Operateur die Leuchte von Hand ausrichtet. Dazu ist zum einen eine aufwendige Mechanik für die Leuchten erforderlich und zum anderen muss der Operateur für jedes Nachstellen der Leuchte seine eigentliche Tätigkeit unterbrechen. Dies verlängert die Operationszeit und stellt damit eine zusätzliche Belastung für den Patienten dar.

Aus dem Stand der Technik ist das Dokument EP 2 495 487 A1, welches als nächstliegender Stand der Technik erachtet wird, bekannt, in welchem eine Beleuchtungsvorrichtung zum Beleuchten einer Operationswunde offenbart ist, bei welcher jeweilige Hauptabstrahlrichtungen von Leuchtmitteln mit einer Tiefenerstreckungsrichtung der Operationswunde in Relation gesetzt werden, um zur Beleuchtung der Operationswunde geeignete Leuchtmittel zu bestimmen. Ferner sei auf das Dokument EP 2 434 202 A1 hingewiesen.

Es ist daher die Aufgabe der vorliegenden Erfindung, eine Beleuchtungsvorrichtung bereitzustellen, welche auf eine vereinfachte Weise eingestellt und, insbesondere automatisch, an sich ändernde Bedingungen während einer Operation angepasst werden kann.

Diese Aufgabe wird durch eine Beleuchtungsvorrichtung zum Beleuchten einer Operationswunde gemäß Anspruch 1 gelöst.

Auf diese Weise kann sowohl erreicht werden, dass Licht, welches von einer Vielzahl von Leuchteinrichtungen emittiert wird, an einem entsprechenden Situs zusammenfällt, so dass eine Beleuchtungsstärke des Situs durch die Beleuchtungsvorrichtung im Vergleich zu einer einzelnen Leuchteinrichtung erhöht werden kann, als auch, dass das Licht derart gerichtet ist, dass es bis zu dem Grund einer schmalen, tiefen Operationswunde reichen kann, um auch dort eine ausreichend hohe Beleuchtungsstärke unter gleichzeitiger Vermeidung von störendem Schattenwurf bereitzustellen. Insbesondere kann so eine homogene Ausleuchtung des Situs erreicht werden.

Es sei bereits an dieser Stelle erwähnt, dass sich der Begriff "emittiertes Licht" je nach Ausbildung der Leuchteinrichtung auf das von der Leuchteinrichtung direkt abgestrahlte Licht oder auf das von einem der Leuchteinrichtung zugeordneten Reflektor reflektierte Licht, insbesondere in Kombination mit dem von der Leuchteinrichtung direkt emittierten Licht, beziehen kann.

Die Hauptabstrahlrichtung kann auf einer Mittelachse eines Leuchtkegels einer jeweiligen Leuchteinrichtung angeordnet sein. Natürlich ist es ebenfalls denkbar, eine entsprechende Mittelachse in das von einer jeweiligen Leuchteinrichtung emittierte Licht zu legen, welches nicht kegelförmig abgestrahlt wird. Vorzugsweise können dabei Schwerpunkte von zueinander parallelen Querschnitten eines von einer jeweiligen Leuchteinrichtung emittierten bzw. von einem einer jeweiligen Leuchteinrichtung zugeordneten Reflektor reflektierten Lichtstrahlenbündels miteinander verbunden werden, um die entsprechende Mittelachse zu bilden.

Der Winkel zwischen einer jeweiligen Hauptabstrahlrichtung und der Tiefenerstreckungsrichtung der Operationswunde kann zwischen einer Projektion einer jeweiligen Hauptabstrahlrichtung und der Tiefenerstreckungsrichtung der Operationswunde in einer Ebene gebildet sein, welche die Tiefenerstreckungsrichtung der Operationswunde und ein Zentrum einer jeweiligen Leuchteinrichtung umfasst. Somit können auch entsprechende Winkelwerte zwischen einer jeweiligen Hauptabstrahlrichtung und der Tiefenerstreckungsrichtung der Operationswunde für den Fall gebildet werden, dass sich die Hauptabstrahlrichtung und die Tiefenerstreckungsrichtung nicht schneiden, das heißt windschief zueinander verlaufen.

Vorteilhafterweise kann der vorbestimmte Bereich des Winkels von 0° bis 75°, insbesondere von 0° bis 60°, besonders bevorzugt von 0° bis 45°, betragen.

Die Leuchteinrichtungen können wenigstens eine LED und/oder wenigstens eine Laserquelle umfassen. Derartige Leuchtquellen sind auf Grund ihrer geringen Wärmeentwicklung bevorzugt, so dass ein in einem Operationssaal herrschendes Klima nicht oder nur geringfügig beeinträchtigt wird.

Vorteilhafterweise kann die Steuerungsvorrichtung dazu eingerichtet sein, nur diejenigen eine mit der Wundöffnungsfläche wenigstens teilweise überlappende beleuchtete Fläche aufweisende Leuchteinrichtungen der Beleuchtungsvorrichtung anzusteuern, welche zwischen ihrer jeweiligen Hauptabstrahlrichtung und der Tiefenerstreckungsrichtung der Operationswunde miteinander einen Winkel einschließen, dessen Wert den vorbestimmten Winkelwert nicht übersteigt. So kann vermieden werden, dass Leuchteinrichtungen, deren jeweilige Hauptabstrahlrichtung den vorbestimmten Winkelwert überschreitet, nicht zur Beleuchtung des Situs beitragen, um einen Intensitätsabfall in der Tiefenerstreckung der Operationswunde zu verhindern.

In einer Weiterbildung der vorliegenden Erfindung kann die Beleuchtungsvorrichtung Luftdurchlässe umfassen, welche dazu eingerichtet sind, einen Luftstrom einer Klimadecke, an welcher die Beleuchtungsvorrichtung angebracht oder in welcher die Beleuchtungsvorrichtung integriert ist, passieren zu lassen. Die Beleuchtungsvorrichtung kann auf diese Weise in Operationssälen installiert werden, ohne den laminaren Luftstrom, welcher üblicherweise in einem Operationsbereich herrscht, zu beeinträchtigen.

Erfindungsgemäß umfassen die Leuchteinrichtungen Licht verschiedener Spektren emittierende Leuchtmittel und sind dazu eingerichtet, durch Mischung des von den Leuchtmitteln emittierten Lichts Licht in einem einstellbaren Spektralbereich zu emittieren. Je nach durchzuführender Operation kann es erwünscht sein, den Spektralbereich der OP-Beleuchtung, beispielsweise in eine eher bläuliche oder eine eher rötliche Beleuchtung, anzupassen. Ferner kann es dabei denkbar sein, alternativ oder zusätzlich zu einer Beleuchtung in einem sichtbaren Spektralbereich eine Beleuchtung des Situs in einem nicht-sichtbaren Spektralbereich, wie beispielsweise einem ultravioletten und/oder infraroten Spektralbereich, zu aktivieren.

Ferner können die Leuchteinrichtungen und/oder einzelne Leuchtmittel der Leuchteinrichtungen in ihrem Lichtstrom einstellbar sein. Es können so beispielsweise einzelne Leuchteinrichtungen gedimmt werden, um eine Beleuchtung von zum Beispiel stark reflektierenden und somit blendenden Bereichen außerhalb des Situs, wodurch ein Operateur bei seiner Tätigkeit beeinträchtigt werden könnte, zu reduzieren.

Die Leuchteinrichtungen können Reflektoren und/oder optische Linsen und/oder optische Konversionsmedien umfassen, welche dazu eingerichtet sind, Licht zu fokussieren und/oder zu streuen und/oder umzulenken und/oder spektral zu verändern. Dabei können die Reflektoren und/oder optischen Linsen, insbesondere unter Verwendung eines Piezoantriebs, relativ zu dem jeweiligen Leuchtmittel verlagerbar und/oder verschwenkbar sein. Piezoantriebe eignen sich insbesondere für die Verlagerung im Sub-Millimeter Bereich, so dass zum Beispiel auch kleinste Fokussierungen einstellbar sein können.

Der Weiteren kann die Beleuchtungsvorrichtung eine Justiereinrichtung umfassen, welche dazu eingerichtet ist, den Winkel zwischen der Hauptabstrahlrichtung wenigstens einer Leuchteinrichtung und der Tiefenerstreckungsrichtung der Operationswunde einzustellen. Dabei kann die Justiereinrichtung der Beleuchtungsvorrichtung das händische Erfassen einer herkömmlichen Operationslampe an deren Handgriff und das Einstellen dieser relativ zu der Operationswunde nachbilden. Die Justiereinrichtung kann hierzu mit der Steuerungsvorrichtung verbunden sein.

Die Justiereinrichtung kann dazu eine Kamera, welche dazu eingerichtet ist, Bereiche, welche sich in der Hauptabstrahlrichtung wenigstens einer Leuchteinrichtung befinden, zu erfassen, und eine Bestimmungseinheit umfassen oder damit verbunden sein, welche auf Grundlage der durch die Kamera erfassten Bereiche dazu eingerichtet ist, eine Operationswunde zu bestimmen, wobei die Justiereinrichtung dazu eingerichtet ist, den Winkel zwischen der Hauptabstrahlrichtung wenigstens einer Leuchteinrichtung und der Tiefenerstreckungsrichtung der Operationswunde, insbesondere automatisch, zu verändern, insbesondere zu verkleinern. Wird beispielsweise eine Beleuchtungsvorrichtung verwendet, welche über eine derart große Anzahl an Leuchteinrichtungen verfügt, dass lediglich eine Auswahl an Leuchteinrichtungen aktiviert werden muss, um die Operationswunde ausreichend zu beleuchten, so kann auch die Justiereinrichtung automatisch diese Auswahl an Leuchteinrichtungen aus allen verfügbaren Leuchteinrichtungen der Beleuchtungsvorrichtung treffen, um den Winkel zwischen einer gemeinsamen Hauptabstrahlrichtung der ausgewählten Leuchteinrichtungen, welche zum Beispiel auf einer Mittelachse oder Schwerpunktachse des Bündels einzelner Hauptabstrahlrichtungen der ausgewählten Leuchteinrichtungen liegen kann, und der Tiefenerstreckungsrichtung der Operationswunde zu minimieren. Beispielsweise können so bei einer Verlagerung des Patienten automatisch diejenigen Leuchteinrichtungen der Beleuchtungsvorrichtung neu ausgewählt werden, welche die Operationswunde optimal ausleuchten.

Ferner kann die Justiereinrichtung eine Kamera, welche dazu eingerichtet ist, Gesten zu erfassen, welche ein Benutzer der Beleuchtungsvorrichtung durchführt, und eine Bestimmungseinheit umfassen oder damit verbunden sein, welche dazu eingerichtet ist, die Gesten vorbestimmten Bedienungsbefehlen zuzuordnen, wobei die Justiereinrichtung dazu eingerichtet ist, eine Ausrichtung einer jeweiligen Hauptabstrahlrichtung von der Justiereinrichtung zugeordneten Leuchteinrichtungen auf Grund der Bedienungsbefehle einzustellen. Beispielsweise kann der Operateur mit einem Finger eine Linie in der Luft ziehen, wodurch die Beleuchtungsvorrichtung veranlasst wird, die einzelnen Hauptabstrahlrichtungen oder die gemeinsame Hauptabstrahlrichtung, wie oben beschrieben, der ausgewählten Leuchteinrichtungen koaxial oder parallel zu dieser Linie auszurichten. Alternativ oder zusätzlich kann die Justiereinrichtung über Spracheingabe, beispielsweise unter Verwendung von akustischen Befehlen durch den Operateur, steuerbar sein.

Die Justiereinrichtung kann auch eine Datenschnittstelle umfassen oder damit verbunden sein, welche dazu eingerichtet ist, Operationsdaten bezüglich einer durchzuführenden Operation an einem Patienten zu empfangen, wobei die Justiereinrichtung dazu eingerichtet ist, eine Ausrichtung einer jeweiligen Hauptabstrahlrichtung von der Justiereinrichtung zugeordneten Leuchteinrichtungen auf Grund der Operationsdaten einzustellen. Die Operationsdaten können beispielsweise selbst und/oder im Zusammenhang mit einer entsprechenden Datenbank anzeigen, in welcher Weise sich eine Tiefenerstreckung eines Situs im Verlauf der Operation ergeben wird. Die Beleuchtungsvorrichtung kann auf dieser Grundlage bereits vor Beginn der Operation diejenigen Leuchteinrichtungen zur Beleuchtung des Situs auswählen, welche auch bei fortgeschrittenem Operationsverlauf für eine Beleuchtung des Situs noch geeignet sind. Es kann somit vermieden werden, dass die Auswahl der Leuchteinrichtungen der Beleuchtungsvorrichtung zur Beleuchtung des Situs während der Operation in einer Weise geändert wird, welche den Operateur, beispielsweise durch wechselnde Schattenbildung, negativ beeinträchtigt.

Beispielsweise kann die Justiereinrichtung ein Eingabegerät umfassen, welches einen stiftartigen Abschnitt umfasst, welcher mit einer Basis verbunden ist, wobei ein Winkel einer Längsachse des stiftartigen Abschnitts zu der Basis veränderbar ist, wobei die Justiereinrichtung dazu eingerichtet sein kann, eine Ausrichtung einer jeweiligen Hauptabstrahlrichtung von der Justiereinrichtung zugeordneten Leuchteinrichtungen auf Grund des Winkels des stiftartigen Abschnitts zu der Basis einzustellen. Das Eingabegerät kann mit der Justiereinrichtung dabei drahtlos, kabelgebunden oder auch über eine Gelenkanordnung verbunden sein.

Dabei kann die Justiereinrichtung dazu eingerichtet sein, eine Ausrichtung einer jeweiligen Hauptabstrahlrichtung von den der Justiereinrichtung zugeordneten Leuchteinrichtungen auf Grund des Winkels des stiftartigen Abschnitts zu der Basis einzeln einzustellen oder eine relative Winkeländerung des Winkels des stiftartigen Abschnitts zu der Basis auf jeden Winkel zwischen einer jeweiligen Hauptabstrahlrichtung von den der Justiereinrichtung zugeordneten Leuchteinrichtungen und der Tiefenerstreckungsrichtung der Operationswunde zu übertragen. Zur Auswahl entsprechender Leuchteinrichtungen, welche der Operateur wünscht, zu justieren, kann das Eingabegerät zusätzlich ein Tastenfeld umfassen, welches beispielsweise der Anordnung von Leuchteinrichtungen an der Beleuchtungsvorrichtung vollständig oder in einer reduzierten Weise entspricht.

Es ist möglich, dass die Justiereinrichtung ein Eingabegerät umfassen kann, welches einen stiftartigen Abschnitt, welcher eine Längsachse aufweist, und eine Erfassungseinheit umfasst, welche dazu eingerichtet ist, die Position und Orientierung der Längsachse des stiftartigen Abschnitts des Eingabegeräts im Raum zu erfassen und an die Justiereinrichtung auszugeben, wobei die Justiereinrichtung dazu eingerichtet sein kann, eine Ausrichtung einer jeweiligen Hauptabstrahlrichtung von der Justiereinrichtung zugeordneten Leuchteinrichtungen auf Grund der erfassten Position und Orientierung der Längsachse des stiftartigen Abschnitts des Eingabegeräts im Raum einzustellen. Das Eingabegerät kann dabei entweder mit der Basis verbunden sein oder von dieser getrennt frei im Raum positionierbar sein. Dabei kann das Eingabegerät einen Entfernungsmesser umfassen, welcher dazu eingerichtet ist, eine Entfernung eines distalen Endes des stiftartigen Abschnitts des Eingabegeräts zu der Operationswunde zu messen und an die Justiereinrichtung auszugeben, wobei die Justiereinrichtung dazu eingerichtet sein kann, eine Fokussierung von der Justiereinrichtung zugeordneten Leuchteinrichtungen auf Grundlage der gemessenen Entfernung einzustellen. Durch ein Entfernen oder Annähern des Eingabegeräts relativ zu dem Situs kann die Beleuchtungsvorrichtung veranlasst werden, dass von den ausgewählten Leuchteinrichtungen emittierte Licht zu defokussieren oder zu fokussieren.

Die Erfassungseinheit kann dabei einen Lagesensor, insbesondere ein Gyroskop, umfassen. Insbesondere kann der Lagesensor zur Erfassung der Position und/oder Orientierung des Eingabegeräts dienen. Der Lagesensor kann dabei in dem stiftartigen Abschnitt des Eingabegeräts angeordnet sein.

Ferner kann die Erfassungseinheit eine Navigationsvorrichtung umfassen, welche dazu eingerichtet ist, eine der Navigationsvorrichtung bekannte Geometrie des Eingabegeräts zu erkennen und in Relation zu der bekannten Geometrie dem Eingabegerät eine Position und Orientierung im Raum zuzuordnen. Die Erfassungseinheit kann beispielsweise derart ausgebildet sein, dass sie Licht, zum Beispiels im Infrarotbereich, aussendet und von dem Eingabegerät reflektiertes Licht empfängt. Aufgrund einer bekannten Relation der Längsachse des Eingabegeräts zu dem durch die Erfassungseinheit erfassten von dem Eingabegerät reflektierten Licht kann die Erfassungseinheit und/oder die Justiereinrichtung die Längsachse des Eingabegeräts im dreidimensionalen Raum bestimmen und entsprechende Befehle zur Ausrichtung einer gemeinsamen oder jeweiligen Hauptabstrahlrichtung der Leuchteinrichtungen an diesen zugeordnete Antriebe ausgeben.

Dabei kann die bekannte Geometrie des Eingabegeräts durch passive oder aktive Marker gebildet sein, deren Lage zu dem stiftartigen Abschnitt des Eingabegeräts fest verbunden oder verbindbar ist. Insbesondere können die passiven Marker als retroreflektierende Marker ausgebildet sein, welche einfallendes Licht gemäß dem Einfallswinkel reflektieren. Aktive Marker hingegen emittieren Licht selbstständig, zum Beispiel unter Verwendung von LEDs.

Ferner kann die Beleuchtungsvorrichtung wenigstens eine Kamera, insbesondere eine Leuchtdichtenkamera, welche eine Beleuchtungsstärke der Operationswunde erfasst, und eine Nachführungseinheit umfassen, welche auf eine durch die Kamera erfasste Abnahme oder Zunahme der Beleuchtungsstärke der Operationswunde hin, die Beleuchtungsvorrichtung derart verändert, dass die Beleuchtungsstärke der Operationswunde konstant bleibt. Die Beleuchtungsvorrichtung kann somit in der Lage sein, eine vorbestimmte Beleuchtungsstärke der Operationswunde auch bei Auftreten einer Abschattung mancher Leuchteinrichtungen in deren Strahlengang zu der Operationswunde hin (bzw. bei Entfernen eines abschattenden Objekts) im Wesentlichen konstant zu halten.

Dabei kann die Nachführungseinheit den Lichtstrom der Leuchteinrichtungen erhöhen, welche eine mit der Wundöffnungsfläche wenigstens teilweise überlappende beleuchtete Fläche aufweisen. Das heißt, diejenigen der ausgewählten und aktivierten Leuchteinrichtungen, welche die Operationswunde bereits vor einem Auftreten einer Abschattung beleuchtet haben und von der Abschattung nicht betroffen sind, beleuchten den Situs nun mit erhöhtem Lichtstrom, so dass die Beleuchtungsstärke der Operationswunde im Wesentlichen der Beleuchtungsstärke vor dem Auftreten der Abschattung entspricht. Dabei könne auch die von der Abschattung betroffenen Leuchteinrichtungen in ihrem Lichtstrom reduziert werden, um einen dadurch entstehenden Schattenwurf zu reduzieren.

Zusätzlich oder alternativ kann die Nachführungseinheit weitere Leuchteinrichtungen der Beleuchtungsvorrichtung aktivieren, deren Hauptabstrahlrichtung mit der Tiefenerstreckungsrichtung der Operationswunde jeweils einen Winkel aufweist, welcher außerhalb des vorbestimmten Bereichs liegt. Das heißt, dass in diesem Fall Leuchteinrichtungen zugeschaltet werden, welche vor dem Auftreten der Abschattung von zur Beleuchtung der Operationswunde verwendeten Leuchteinrichtungen nicht zur Beleuchtung der Operationswunde verwendet worden sind. Die Auswahl von zusätzlichen Leuchteinrichtungen, welche zur Beleuchtung der Operationswunde aktiviert werden sollen, kann zum Beispiel anhand des Winkels von deren Hauptabstrahlrichtung mit der Tiefenerstreckungsrichtung der Operationswunde priorisiert werden. Allgemein wird es von Operateuren bevorzugt, zusätzliche Leuchteinrichtungen zu aktivieren, obwohl deren Winkel mit der Tiefenerstreckungsrichtung der Operationswunde außerhalb des vorbestimmten Bereichs liegen, als an einem zu gering beleuchteten Situs zu arbeiten.

Hierbei kann die Nachführungseinheit beim Aktivieren von weiteren Leuchteinrichtungen mit denjenigen Leuchteinrichtungen beginnen, welche den geringsten Winkel zwischen ihrer Hauptabstrahlrichtung mit der Tiefenerstreckungsrichtung der Operationswunde aufweisen. Somit kann gewährleistet werden, dass weiterhin eine gute Tiefenausleuchtung der Operationswunde erreicht werden kann.

Ferner kann die Nachführungseinheit eine weitere Anordnungsgruppe mit Leuchteinrichtungen aktivieren, welche von der Anordnungsgruppe der Beleuchtungsvorrichtung getrennt angeordnet ist. Insbesondere kann eine Ebene, in welcher sich die Leuchteinrichtungen der weiteren Anordnungsgruppe erstrecken, zu einer Ebene, in welcher sich die Leuchteinrichtungen der oben erwähnten Anordnungsgruppe erstrecken, gewinkelt angeordnet sein. Beispielsweise kann, je nach Art der Abschattung der Leuchteinrichtungen, die weitere Anordnungsgruppe derart aktiviert werden, dass ein Objekt, welches die Abschattung hervorruft, nicht mehr auf dem Weg des Lichts zwischen einer jeweiligen Leuchteinrichtung und der Operationswunde liegt, so dass dieses sozusagen durch die Leuchteinrichtungen der weiteren Anordnungsgruppe "umleuchtet" werden kann.

Vorteilhafterweise kann die Nachführungseinheit wenigstens einen Leuchtkegel einer Leuchteinrichtung fokussieren. Auch durch das Fokussieren von jeweiligen Leuchtkegeln kann eine Beleuchtungsstärke einer Operationswunde erhöht werden, falls dies notwendig ist.

Ferner kann die Beleuchtungsvorrichtung eine Mehrzahl von Kameras umfassen, welche eine Beleuchtungsstärke der Operationswunde erfassen, und welche derart angeordnet sind, dass deren Sichtachsen auf die Operationswunde zueinander einen vorbestimmten Winkel bilden, welcher dazu geeignet ist, eine Verdeckung einer Sicht einer ersten Kamera auf die Operationswunde durch eine zweite Kamera zu kompensieren. Es kann dadurch gewährleistet werden, dass das automatische Nachführen bzw. Anpassen der Beleuchtungsstärke der Operationswunde selbst dann realisierbar ist, wenn eine der Kameras, welche eine Beleuchtungsstärke der Operationswunde erfasst, keine freie Sicht auf die Operationswunde hat.

Die Beleuchtungsvorrichtung kann ferner eine Abstandsmesseinrichtung umfassen, welche einen Abstand der Beleuchtungsvorrichtung zu der Operationswunde misst, wobei die Beleuchtungsvorrichtung jeweilige Leuchteinrichtungen auf Grundlage der Abstandsmessung auf die Operationswunde fokussiert. Die Beleuchtungsvorrichtung kann auf diese Weise automatisch auf sich ändernde Operationsbedingungen, wie beispielsweise das Heben und Senken eines Operationstisches, reagieren und eine gleichbleibende Beleuchtung der Operationswunde gewährleisten.

Ferner kann die Beleuchtungsvorrichtung eine automatische Fokussierung von Leuchteinrichtungen durchführen, bis ein Anteil des beleuchteten Felds einer jeweiligen Leuchteinrichtung, welcher mit der Operationswunde überlappt, relativ zu dem gesamten beleuchteten Feld maximiert ist. Insbesondere können so Bereiche des Leuchtfeldes reduziert werden, welche eine Umgebung des Situs beleuchten, in welcher üblicherweise ein Abdecktuch angeordnet ist, welches aufgrund seiner starken Reflexion möglichst nicht stark beleuchtet werden soll.

Die Beleuchtungsvorrichtung kann eine Mehrzahl von Kameras, welche zumindest die Operationswunde erfassen und eine Kombinationseinheit umfassen, wobei die von der Mehrzahl von Kameras erfassten Perspektiven der Operationswunde bzw. der gesamten Operationsumgebung von der Kombinationseinheit zu einem dreidimensionalen Modell kombiniert werden. Dabei kann die Beleuchtungsvorrichtung auf Grundlage des dreidimensionalen Modells eine Hauptabstrahlrichtung wenigstens einer Leuchteinrichtung identifizieren, deren Licht durch ein Hindernis, welches zwischen der Beleuchtungsvorrichtung und der Operationswunde angeordnet ist, zumindest teilweise daran gehindert wird, auf die Operationswunde zu treffen, und/oder eine Hauptabstrahlrichtung wenigstens einer Leuchteinrichtung identifiziert, deren Licht trotz eines Hindernisses, welches zwischen der Beleuchtungsvorrichtung und der Operationswunde angeordnet ist, auf die Operationswunde trifft. Durch die Verwendung des dreidimensionalen Modells kann eine Lage und/oder Größe des Objekts, welches, wie oben beschrieben, eine Abschattung hervorruft, im Raum eindeutig bestimmt werden, so dass sich Leuchteinrichtungen und gegebenenfalls vorzunehmende Anpassungen an der Beleuchtungsvorrichtung identifizieren lassen, um das Objekt, wie zuvor erwähnt, zu "umleuchten".

Dabei kann die Beleuchtungsvorrichtung auf Grundlage des dreidimensionalen Modells und der wenigstens einen identifizierten Hauptabstrahlrichtung wenigstens eine Leuchteinrichtung verändern, insbesondere aktivieren oder deaktivieren, um eine Beleuchtungsstärke der Operationswunde zu steigern oder abzusenken. Insbesondere in dem Fall, in welchem Objekte, welche in den Strahlengang wenigstens einer Leuchteinrichtung eingeführt werden, eine Abschattung und somit eine Verringerung der Beleuchtungsintensität der Wunde hervorrufen, können diese, an der Abschattung beteiligten, Leuchteinrichtungen in ihrer Intensität vermindert oder gar deaktiviert werden und wenigstens eine der restlichen, derzeit aktivierten, Leuchteinrichtungen in ihrer Intensität gesteigert und/oder wenigstens eine zusätzliche Leuchteinrichtung aktiviert werden.

Die Beleuchtungsvorrichtung kann auch eine Abstandsmesseinrichtung und/oder eine Kamera umfassen, welche ein sich über die Operationswunde bewegendes Objekt erfassen/erfasst, wobei auf Grundlage des erfassten Objekts eine Veränderung der Beleuchtungseinrichtung durchgeführt werden kann, um eine Abschattung der Operationswunde durch das Objekt zu minimieren. Es kann dadurch eine direkte Korrelation zwischen dem sich bewegenden Objekt, welches eine Abschattung hervorruft, und der Anpassung der Beleuchtungseinrichtung hergestellt werden. Hierdurch kann der benötigte Aufwand für eine genaue und schnell reagierende Anpassung der Beleuchtungsvorrichtung reduziert werden.

Nachfolgend wird ein Verfahren zum Beleuchten einer Operationswunde beschrieben, wobei das Verfahren die Schritte umfasst:
Bereitstellen einer Beleuchtungsvorrichtung, insbesondere einer erfindungsgemäßen Beleuchtungsvorrichtung, welche eine Mehrzahl von Leuchteinrichtungen aufweist, welche jeweils dazu eingerichtet sind, Licht in einer Hauptabstrahlrichtung zu emittieren, wobei wenigstens manche der Leuchteinrichtungen eine Anordnungsgruppe bilden, in welcher die Leuchteinrichtungen in einer im Wesentlichen unveränderlichen Position in Bezug auf ein Raumkoordinatensystem angeordnet sind,
Eingeben von Informationen über eine Position einer Operationswunde in dem Raumkoordinatensystem, welche an der Oberfläche des Körpers eines Patienten eine Wundenöffnungsfläche aufweist,
Bestimmen einer Tiefenerstreckungsrichtung der Wunde in den Körper des Patienten hinein,
Bestimmen wenigstens einer Leuchteinrichtung der Beleuchtungsvorrichtung, welche dazu eingerichtet ist, Licht derart zu emittieren, dass eine von dieser wenigstens einen Leuchteinrichtung beleuchtete Fläche mit der Wundenöffnungsfläche wenigstens teilweise überlappt, und dass deren Hauptabstrahlrichtung mit der Tiefenerstreckungsrichtung der Operationswunde einen Winkel einschließt, dessen Wert einen vorbestimmten Winkelwert nicht übersteigt, und
Aktivieren der derart bestimmten Leuchteinrichtungen, um die Operationswunde zu beleuchten.

Es sei bereits an dieser Stelle darauf hingewiesen, dass sämtliche für die erfindungsgemäße Beleuchtungsvorrichtung beschriebene Merkmale, Effekte und Vorteile ebenso für das Verfahren Anwendung finden können, und umgekehrt.

Das Verfahren kann ferner umfassen:
Einstellen des Winkels zwischen der Hauptabstrahlrichtung wenigstens einer Leuchteinrichtung und der Tiefenerstreckungsrichtung der Operationswunde durch Verschwenken der jeweiligen Leuchteinrichtung. Dies kann, wie in Bezug auf die Vorrichtung dargelegt, zum Beispiel unter Verwendung einer Justiereinrichtung geschehen.

Das Verfahren kann ein Verändern des Lichtstroms und/oder des Emissionsspektrums von wenigstens einer der Leuchteinrichtungen umfassen. Auf diese Weise kann eine Beleuchtungsintensität an eine momentane Operationssituation angepasst werden. Es ist auch denkbar, dass eine Intensitätssteuerung der Leuchteinrichtungen in Abhängigkeit des vorbestimmten Winkelwertes zwischen Hauptabstrahlrichtung und Tiefenerstreckungsrichtung durchgeführt wird.

Insbesondere kann das Verfahren ferner umfassen:
Erfassen von Bereichen, welche sich in der Hauptabstrahlrichtung wenigstens einer Leuchteinrichtung befinden, durch eine Kamera, Bestimmen von Informationen über die Position der Operationswunde in dem Raumkoordinatensystem auf Grundlage der durch die Kamera erfassten Bereiche,
Einstellen, insbesondere Verkleinern, des Winkels zwischen der Hauptabstrahlrichtung wenigstens einer Leuchteinrichtung und der Tiefenerstreckungsrichtung der Operationswunde, insbesondere automatisch.

Vorteilhafterweise kann das Verfahren ferner umfassen:
Bereitstellen einer Datenschnittstelle, über welche Daten an die Beleuchtungsvorrichtung weitergegeben werden,
Eingeben von Operationsdaten, auf Grund welcher eine grundlegende Vorhersage der Position der Operationswunde in dem Raumkoordinatensystem möglich ist, insbesondere präoperativ, über die Datenschnittstelle, um eine Ausrichtung einer jeweiligen Hauptabstrahlrichtung wenigstens einer Leuchteinrichtung auf Grundlage der Operationsdaten einzustellen.

Im Folgenden wird die vorliegende Erfindung in größerem Detail anhand eines Ausführungsbeispiels mit Bezug auf die begleitenden Zeichnungen beschrieben werden. Es stellt dar:
- Figur 1: eine perspektivische Ansicht einer Operationssituation, wobei eine Operationswunde von einer erfindungsgemäßen Beleuchtungsvorrichtung beleuchtet wird; und
- Figur 2: einen Detailausschnitt aus Figur 1.

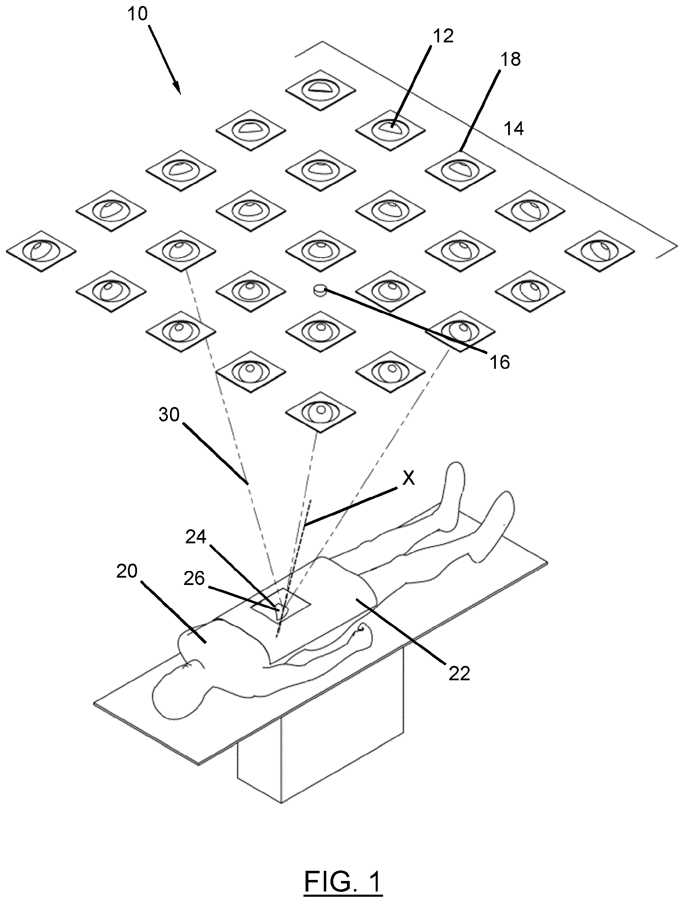

In Figur 1 ist eine erfindungsgemäße Beleuchtungsvorrichtung allgemein mit dem Bezugszeichen 10 bezeichnet. Die Beleuchtungsvorrichtung 10 umfasst hier eine Mehrzahl von Leuchteinrichtungen 12, welche gemeinsam eine Anordnungsgruppe 14 bilden, und eine Kamera 16. Die einzelnen Leuchteinrichtungen 12 sind in der in Figur 1 dargestellten Anordnungsgruppe 14 in einer quadratischen Konfiguration von fünfundzwanzig Leuchteinrichtungen 12 und zueinander gleichbeabstandet angeordnet, so dass Luft, beispielsweise aus einer Klimadecke des Operationsraums, zwischen den einzelnen Leuchteinrichtungen 12 hindurchtreten kann. Jede der Leuchteinrichtungen 12 umfasst wenigstens ein in Figur 1 nicht näher dargestelltes Leuchtmittel, beispielsweise in Form wenigstens einer LED. Dabei ist die generelle Anordnung der Leuchteinrichtungen 12 jedoch nicht auf die in Figur 1 dargestellte Variante beschränkt, so dass auch eine andere Anzahl und/oder andere Konfiguration von Leuchteinrichtungen 12 möglich ist, welche beispielsweise elliptisch oder rechteckig ist.

Jedes Leuchtmittel einer Leuchteinrichtung 12 ist, insbesondere in Kombination mit einem zugehörigen Reflektor, schwenkbar zu einer Halterung 18 der Leuchteinrichtung 12 gelagert, wobei die jeweilige Leuchteinrichtung 12 über die Halterung 18 mit der Decke des Operationsraums verbunden ist. Zur Ansteuerung einer Verschwenkung des Leuchtmittels relativ zu der Halterung 18 kann insbesondere wenigstens ein Elektromotor vorgesehen sein, welcher mit einer Steuerungsvorrichtung verbunden ist und dazu eingerichtet ist, das zugehörige Leuchtmittel relativ zu der Halterung 18 zu verlagern.

In Figur 1 ist ferner ein Patient 20 zu sehen. Im Rahmen einer an dem Patienten 20 durchzuführenden Operation wird der Patient mit einem Abdecktuch 22 bedeckt, wobei eine zu behandelnde Stelle (auch "Operationswunde" genannt) 24 an dem Körper des Patienten 22 frei verbleibt. Die Operationswunde 24 weist an der Haut des Patienten 22 eine Wundenöffnungsfläche 26 und entlang des in den Körper des Patienten 22 hinein verlaufenden Wundkanals eine Tiefenerstreckungsrichtung X auf.

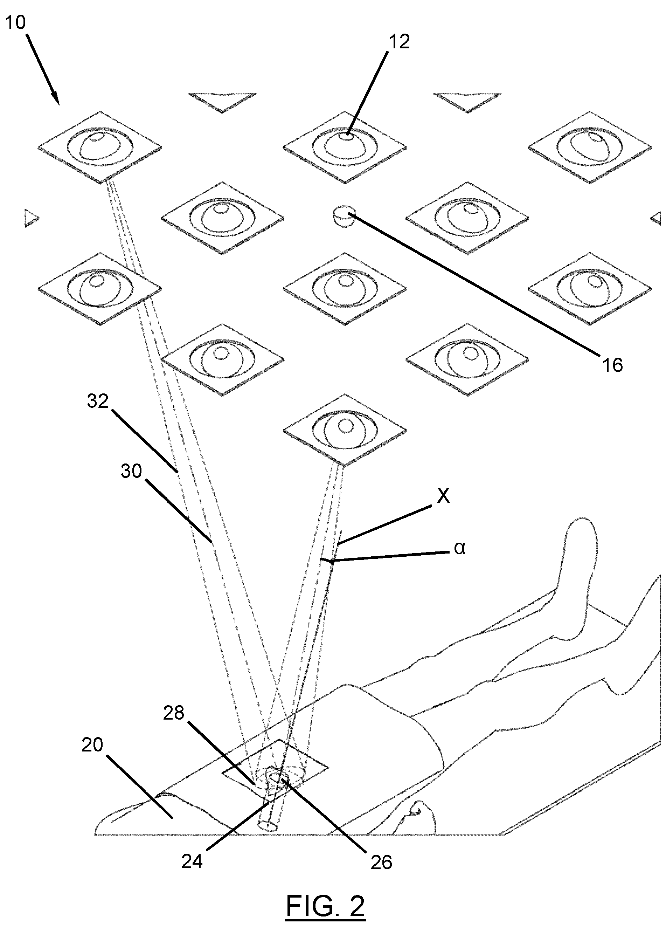

Damit ein Operateur den Patienten angemessen behandeln kann, muss die Operationswunde 24 ausreichend beleuchtet werden. Zu diesem Zweck werden Leuchteinrichtungen 12 aktiviert und so justiert, dass eine durch eine jeweilige Leuchteinrichtung 12 beleuchtete Fläche 28 (s. Fig. 2) zumindest teilweise mit der Wundenöffnungsfläche 26 überlappt. Jeder Leuchteinrichtung 12 kann dabei eine Hauptabstrahlrichtung 30 zugewiesen werden. Mit Bezug auf Figur 2 ist zu erkennen, dass eine jeweilige Hauptabstrahlrichtung 30 die Mittelachse eines hier von dem Leuchtmittel zu der Wundenöffnungsfläche 26 konisch verlaufenden Leuchtkegels 32 sein kann. Es werden zur Beleuchtung der Operationswunde 24 nur diejenigen Leuchteinrichtungen 12 ausgewählt, welche in einer entsprechenden Justierung eine mit der Wundöffnungsfläche 26 wenigstens teilweise überlappende beleuchtete Fläche 28 aufweisen und deren jeweilige Hauptabstrahlrichtung 30 mit der Tiefenerstreckungsrichtung X der Operationswunde 24 einen vorbestimmten Winkelwert, welcher vorzugsweise zwischen 0° und 45° liegt, nicht übersteigt. In Figur 2 ist beispielhaft ein derartiger Winkel zwischen einer Hauptabstrahlrichtung 30 und der Tiefenerstreckungsrichtung X der Operationswunde 24 mit α bezeichnet. Hierdurch kann gewährleistet werden, dass die Operationswunde 24 auch in ihrer gesamten Tiefe gut beleuchtet wird.

Den zur Beleuchtung der Operationswunde 24 aktivierten Leuchteinrichtungen 12 kann auch eine gemeinsame Hauptabstrahlrichtung zugewiesen werden, welche sich beispielsweise entlang einer Mittelachse der Summe aller entsprechenden Leuchtkegel erstreckt. Über eine Justiereinrichtung (nicht dargestellt) kann die gemeinsame Hauptabstrahlrichtung und/oder jeweilige Hauptabstrahlrichtungen 30 verändert werden, um die Beleuchtung an eine sich verändernde Operationssituation anzupassen. So können die Strahlengänge der Leuchteinrichtungen 12 an der Wundenöffnungsfläche 26 zum Beispiel fokussiert werden. Im Zuge einer derartigen Anpassung können auch Leuchteinrichtungen 12 deaktiviert oder in ihrem Lichtstrom verändert werden und/oder es können bislang nicht verwendete Leuchteinrichtungen 12 aktiviert werden. Um Reflexionen zu vermeiden, welche von dem Abdecktuch 22 ausgehen können, können die Leuchteinrichtungen 12 derart eingestellt sein, dass das Abdecktuch 22 möglichst nicht beleuchtet wird.

Die Kamera 16, welche sich in dem hier dargestellten Ausführungsbeispiel in unmittelbarer Nähe zu einer zentralen Leuchteinrichtung 12 befindet, jedoch ebenso in einem äußeren Bereich der Beleuchtungsvorrichtung 10 angeordnet sein könnte, ist dazu eingerichtet, den Bereich unterhalb der Mehrzahl von Leuchteinrichtungen 12 zu überwachen.

Die Kamera 16 ist hier dazu eingerichtet, sowohl den Bereich einer Operationswunde 24 zu erfassen, wodurch ein zu beleuchtender Bereich automatisch bestimmt werden kann, als auch Gesten eines Operateurs zu erkennen, um dadurch über die Steuerungsvorrichtung und die Justiereinrichtung die Ausrichtung der Leuchteinrichtungen 12 zu verändern, Objekte zu erfassen, welche sich zwischen der Beleuchtungsvorrichtung 10 und der Operationswunde 24 befinden, um so zu bestimmen, ob bzw. welche Leuchteinrichtungen 12 in ihrem Lichtstrom verändert werden sollen, und Änderungen der Beleuchtungsstärke der Operationswunde 24 zu bestimmen, so dass die Beleuchtungsstärke der Operationswunde 24 möglichst konstant bleibt.

Sowohl der Tiefenerstreckungsrichtung X als auch den Hauptabstrahlrichtungen 30 der Leuchteinrichtungen 12 können Koordinaten bzw. Vektoren in einem Raumkoordinatensystem X, Y, Z zugewiesen werden. Hierdurch können beispielsweise die Winkel zwischen den Hauptabstrahlrichtungen 30 und der Tiefenerstreckungsrichtung X unabhängig von einer Projektionsebene bestimmt werden.

Die Leuchteinrichtungen 12, welche die Anordnungsgruppe 14 bilden, sind derart an der Decke des Operationssaals angebracht, dass sie in einer zueinander unveränderlichen Position, jedoch veränderbaren Orientierung (Ausrichtung des jeweiligen Leuchtkegels) angeordnet sind.

Die Beleuchtungsvorrichtung 10 ermöglicht somit eine gute Anpassung an beliebige Operationssituationen, wobei dadurch, dass die Beleuchtungsvorrichtung 10 im Wesentlichen nicht von der Decke des Operationssaals vorsteht, die unmittelbare Umgebung über dem Patienten besser durch den Operateur bzw. sonstiges Operationsequipment genutzt werden kann.

## Patentansprüche

1. Beleuchtungsvorrichtung (10) zum Beleuchten einer Operationswunde (24), welche an der Oberfläche des Körpers eines Patienten (20) eine Wundenöffnungsfläche (26) aufweist und sich in einer Tiefenerstreckungsrichtung in den Körper des Patienten (20) hinein erstreckt,
wobei die Beleuchtungsvorrichtung (10) eine Steuerungsvorrichtung umfasst mit Mitteln zur Eingabe von Informationen über eine Position der Wundenöffnungsfläche (26) und die Tiefenerstreckungsrichtung (X) in einem Raumkoordinatensystem,
wobei die Beleuchtungsvorrichtung (10) eine Mehrzahl von Leuchteinrichtungen (12) aufweist, welche jeweils dazu eingerichtet sind, Licht in einer Hauptabstrahlrichtung (30) zu emittieren, und welche jeweils wenigstens ein Leuchtmittel aufweisen,
wobei wenigstens eine der Leuchteinrichtungen (12) dazu eingerichtet ist, Licht derart zu emittieren, dass eine von dieser wenigstens einen Leuchteinrichtung (12) beleuchtete Fläche (28) mit der Wundenöffnungsfläche (26) wenigstens teilweise überlappt,
wobei die Hauptabstrahlrichtung (30) jeder Leuchteinrichtung (12), welche eine mit der Wundöffnungsfläche (26) wenigstens teilweise überlappende beleuchtete Fläche (28) aufweist, und die Tiefenerstreckungsrichtung (X) der Operationswunde (24) miteinander jeweils einen Winkel einschließen, dessen Wert einen vorbestimmten Winkelwert nicht übersteigt,
wobei wenigstens diejenigen Leuchteinrichtungen (12), welche eine mit der Wundöffnungsfläche (26) wenigstens teilweise überlappende beleuchtete Fläche (28) aufweisen und deren jeweilige Hauptabstrahlrichtung (30) mit der Tiefenerstreckungsrichtung (X) der Operationswunde (24) einen vorbestimmten Winkelwert nicht übersteigt, eine Anordnungsgruppe (14) bilden, wobei die Leuchteinrichtungen (12) in der Anordnungsgruppe in einer im Wesentlichen unveränderlichen Position in Bezug auf das Raumkoordinatensystem angeordnet sind **dadurch gekennzeichnet, dass** die Leuchteinrichtungen (12) Licht verschiedener Spektren emittierende Leuchtmittel umfassen und dazu eingerichtet sind, durch Mischung des von den Leuchtmitteln emittierten Lichts Licht in einem einstellbaren Spektralbereich zu emittieren.

2. Beleuchtungsvorrichtung (10) nach Anspruch 1,
**dadurch gekennzeichnet, dass** die Steuerungsvorrichtung dazu eingerichtet ist, nur diejenigen eine mit der Wundöffnungsfläche (26) wenigstens teilweise überlappende beleuchtete Fläche (28) aufweisende Leuchteinrichtungen (12) der Beleuchtungsvorrichtung (10) anzusteuern, welche zwischen ihrer jeweiligen Hauptabstrahlrichtung (30) und der Tiefenerstreckungsrichtung (X) der Operationswunde miteinander einen Winkel einschließen, dessen Wert den vorbestimmten Winkelwert nicht übersteigt.

3. Beleuchtungsvorrichtung (10) nach einem der vorhergehenden Ansprüche,
**dadurch gekennzeichnet, dass** die Beleuchtungsvorrichtung (10) Luftdurchlässe umfasst, welche dazu eingerichtet sind, einen Luftstrom einer Klimadecke, an welcher die Beleuchtungsvorrichtung (10) angebracht oder in welcher die Beleuchtungsvorrichtung (10) integriert ist, passieren zu lassen.

4. Beleuchtungsvorrichtung (10) nach einem der vorhergehenden Ansprüche,
**dadurch gekennzeichnet, dass** die Leuchteinrichtungen (12) und/oder einzelne Leuchtmittel der Leuchteinrichtungen (12) in ihrem Lichtstrom einstellbar sind.

5. Beleuchtungsvorrichtung (10) nach einem der vorhergehenden Ansprüche,
**dadurch gekennzeichnet, dass** die Leuchteinrichtungen (12) Reflektoren und/oder optische Linsen und/oder optische Konversionsmedien umfassen, welche dazu eingerichtet sind, Licht zu fokussieren und/oder zu streuen und/oder umzulenken und/oder spektral zu verändern.

6. Beleuchtungsvorrichtung (10) nach einem der vorhergehenden Ansprüche,
**dadurch gekennzeichnet, dass** die Beleuchtungsvorrichtung (10) eine Justiereinrichtung umfasst, welche dazu eingerichtet ist, den Winkel zwischen der Hauptabstrahlrichtung (30) wenigstens einer Leuchteinrichtung (12) und der Tiefenerstreckungsrichtung (X) der Operationswunde (24) einzustellen.

7. Beleuchtungsvorrichtung (10) nach dem vorhergehenden Anspruch,
**dadurch gekennzeichnet, dass** die Justiereinrichtung eine Kamera (16), welche dazu eingerichtet ist, Bereiche, welche sich in der Hauptabstrahlrichtung (30) wenigstens einer Leuchteinrichtung (12) befinden, zu erfassen, und eine Bestimmungseinheit umfasst oder damit verbunden ist, welche auf Grundlage der durch die Kamera (16) erfassten Bereiche dazu eingerichtet ist, eine Operationswunde (24) zu bestimmen,
wobei die Justiereinrichtung dazu eingerichtet ist, den Winkel zwischen der Hauptabstrahlrichtung wenigstens einer Leuchteinrichtung (12) und der Tiefenerstreckungsrichtung (X) der Operationswunde (24), insbesondere automatisch, zu verändern, insbesondere zu verkleinern.

8. Beleuchtungsvorrichtung (10) nach Anspruch 6 oder 7,
**dadurch gekennzeichnet, dass** die Justiereinrichtung eine Kamera (16), welche dazu eingerichtet ist, Gesten zu erfassen, welche ein Benutzer der Beleuchtungsvorrichtung durchführt, und eine Bestimmungseinheit umfasst oder damit verbunden ist, welche dazu eingerichtet ist, die Gesten vorbestimmten Bedienungsbefehlen zuzuordnen,
wobei die Justiereinrichtung dazu eingerichtet ist, eine Ausrichtung einer jeweiligen Hauptabstrahlrichtung (30) von der Justiereinrichtung zugeordneten Leuchteinrichtungen (12) auf Grund der Bedienungsbefehle einzustellen.

9. Beleuchtungsvorrichtung (10) nach einem der Ansprüche 6 bis 8,
**dadurch gekennzeichnet, dass** die Justiereinrichtung eine Datenschnittstelle umfasst oder damit verbunden ist, welche dazu eingerichtet ist, Operationsdaten bezüglich einer durchzuführenden Operation an einem Patienten zu empfangen,
wobei die Justiereinrichtung dazu eingerichtet ist, eine Ausrichtung einer jeweiligen Hauptabstrahlrichtung (30) von der Justiereinrichtung zugeordneten Leuchteinrichtungen (12) auf Grund der Operationsdaten einzustellen.

## Claims

1. An illumination device (10) for illuminating a surgical wound (24), which has a wound opening area (26) on a surface of a body of a patient (20) and which extends in a depth extension direction into the body of the patient (20),
wherein the illumination device (10) comprises a control device with means for inputting information about a position of the wound opening area (26) and the depth extension direction (X) in a spatial coordinate system,
wherein the illumination device (10) has a plurality of lighting devices (12) which are each configured to emit light in a main emission direction (30) and which each have at least one illuminant,
wherein at least one of the lighting devices (12) is configured to emit light such that an area (28) illuminated by said at least one lighting device (12) at least partially overlaps the wound opening area (26),
wherein the main emission direction (30) of each lighting device (12), which has an illuminated area (28) at least partially overlapping with the wound opening area (26), and the depth extension direction (X) of the surgical wound (24) each enclose an angle with one another whose value does not exceed a predetermined angle value,
wherein at least those lighting devices (12) that have an illuminated area (28) at least partially overlapping the wound opening area (26) and whose respective main emission direction (30) does not exceed a predetermined angular value with the depth extension direction (X) of the surgical wound (24) form an array (14), wherein the lighting devices (12) in the array are arranged in a substantially unchangeable position with respect to the spatial coordinate system, **characterized in that** the lighting devices (12) comprise illuminants emitting light of different spectra and are configured to emit light in an adjustable spectral range by mixing the light emitted by the illuminants.

2. The illumination device (10) according to claim 1,
**characterized in that** the control device is configured to control only those lighting devices (12) of the illumination device (10) that have an illuminated area (28) at least partially overlapping the wound opening area (26), which lighting devices (12), between their respective main emission direction (30) and the depth extension direction (X) of the surgical wound, enclose an angle whose value does not exceed the predetermined angle value.

3. The illumination device (10) according to any one of the preceding claims,
**characterized in that** the illumination device (10) comprises air passages which are configured to allow an air flow of an air-conditioning ceiling, to which the illumination device (10) is attached or in which the illumination device (10) is integrated, to pass.

4. The illumination device (10) according to any one of the preceding claims,
**characterized in that** the lighting devices (12) and/or individual illuminants of the lighting devices (12) can be set in their luminous flux.

5. The illumination device (10) according to any one of the preceding claims,
**characterized in that** the lighting devices (12) comprise reflectors and/or optical lenses and/or optical conversion media which are configured to focus and/or scatter and/or deflect light and/or to change light spectrally.

6. The illumination device (10) according to any one of the preceding claims,
**characterized in that** the illumination device (10) comprises an adjusting device which is configured to adjust the angle between the main emission direction (30) of at least one lighting device (12) and the depth extension direction (X) of the surgical wound (24).

7. The illumination device (10) according to the preceding claim,
**characterized in that** the adjusting device comprises a camera (16) which is configured to detect regions which are located in the main emission direction (30) of at least one lighting device (12), and a determination unit, or is connected thereto, which is configured, on the basis of the regions detected by the camera (16), to determine a surgical wound (24),
wherein the adjusting device is configured, in particular automatically, to change, in particular to reduce, the angle between the main emission direction of at least one lighting device (12) and the depth extension direction (X) of the surgical wound (24).

8. The illumination device (10) according to claim 6 or 7,
**characterized in that** the adjusting device comprises a camera (16) which is configured to detect gestures which a user of the illumination device carries out, and a determination unit, or is connected thereto, which is configured to assign the gestures to predetermined operating commands,
wherein the adjusting device is configured to adjust an alignment of a respective main emission direction (30) of lighting devices (12) associated with the adjusting device on the basis of the operating commands.

9. The illumination device (10) according to any one of claims 6 to 8,
**characterized in that** the adjusting device comprises or is connected to a data interface which is configured to receive surgery data relating to a surgery to be carried out on a patient,
wherein the adjusting device is configured to adjust an alignment of a respective main emission direction (30) of lighting devices (12) associated with the adjusting device on the basis of the surgery data.

## Revendications

1. Dispositif d'éclairage (10) destiné à éclairer une plaie chirurgicale (24) qui présente, à la surface du corps d'un patient (20), une surface d'ouverture de plaie (26) et qui s'étend dans le corps du patient (20) selon une direction d'extension en profondeur, dans lequel le dispositif d'éclairage (10) comprend un dispositif de commande doté de moyens permettant de saisir des informations relatives à la position de la surface d'ouverture de plaie (26) et à la direction d'extension en profondeur (X) dans un système de coordonnées spatiales,
dans lequel le dispositif d'éclairage (10) comporte une pluralité de dispositifs lumineux (12) qui sont chacun agencés pour émettre de la lumière dans une direction principale de rayonnement (30), et qui comportent chacun au moins un moyen d'éclairage,
dans lequel au moins l'un des dispositifs lumineux (12) est agencé pour émettre de la lumière de telle sorte qu'une surface éclairée (28) par ce au moins un dispositif lumineux (12) chevauche au moins partiellement la surface d'ouverture de plaie (26),
dans lequel la direction principale de rayonnement (30) de chaque dispositif lumineux (12) présentant une surface éclairée (28) qui chevauche au moins partiellement la surface d'ouverture de plaie (26) et la direction d'extension en profondeur (X) de la plaie chirurgicale (24) forment entre elles respectivement un angle dont la valeur ne dépasse pas une valeur angulaire prédéterminée,
dans lequel au moins les dispositifs lumineux (12) qui présentent une surface éclairée (28) chevauchant au moins partiellement la surface d'ouverture de plaie (26) et dont la direction principale de rayonnement (30) respective ne dépasse pas une valeur angulaire prédéterminée par rapport à la direction d'extension en profondeur (X) de la plaie chirurgicale (24), forment un groupe d'agencement (14), les dispositifs lumineux (12) du groupe d'agencement étant disposés dans une position sensiblement invariable par rapport au système de coordonnées spatiales, **caractérisé en ce que** les dispositifs lumineux (12) comprennent des moyens d'éclairage émettant de la lumière de différents spectres et sont agencés pour émettre, par mélange de la lumière émise par les moyens d'éclairage, de la lumière dans une plage spectrale réglable.

2. Dispositif d'éclairage (10) selon la revendication 1,
**caractérisé en ce que** le dispositif de commande est agencé pour n'activer que les dispositifs lumineux (12) du dispositif d'éclairage (10) qui présentent une surface éclairée (28) chevauchant au moins partiellement la surface d'ouverture de plaie (26) et dont les directions de rayonnement principales (30) respectives forment entre elles, par rapport à la direction d'extension en profondeur (X) de la plaie chirurgicale, un angle dont la valeur ne dépasse pas la valeur angulaire prédéterminée.

3. Dispositif d'éclairage (10) selon l'une des revendications précédentes,
**caractérisé en ce que** le dispositif d'éclairage (10) comprend des passages d'air qui sont agencés pour laisser passer un flux d'air provenant d'un plafond climatisé sur lequel le dispositif d'éclairage (10) est monté ou dans lequel le dispositif d'éclairage (10) est intégré.

4. Dispositif d'éclairage (10) selon l'une des revendications précédentes,
**caractérisé en ce que** le flux lumineux des dispositifs lumineux (12) et/ou de certains moyens d'éclairage des dispositifs lumineux (12) est réglable.

5. Dispositif d'éclairage (10) selon l'une des revendications précédentes,
**caractérisé en ce que** les dispositifs lumineux (12) comprennent des réflecteurs et/ou des lentilles optiques et/ou des milieux de conversion optique, qui sont agencés pour focaliser et/ou diffuser et/ou dévier et/ou modifier spectralement la lumière.

6. Dispositif d'éclairage (10) selon l'une des revendications précédentes,
**caractérisé en ce que** le dispositif d'éclairage (10) comprend un dispositif de réglage qui est agencé pour ajuster l'angle entre la direction principale de rayonnement (30) d'au moins un dispositif lumineux (12) et la direction d'extension en profondeur (X) de la plaie chirurgicale (24).

7. Dispositif d'éclairage (10) selon la revendication précédente,
**caractérisé en ce que** le dispositif de réglage comprend une caméra (16), qui est agencée pour détecter des zones situées dans la direction principale de rayonnement (30) d'au moins un dispositif lumineux (12), et comprend une unité de détermination, ou est relié à celle-ci, qui est agencée sur la base des zones détectées par la caméra (16) pour déterminer une plaie chirurgicale (24), dans lequel le dispositif de réglage est agencé pour modifier, notamment automatiquement, l'angle entre la direction principale de rayonnement d'au moins un dispositif lumineux (12) et la direction d'extension en profondeur (X) de la plaie chirurgicale (24), notamment pour le réduire.

8. Dispositif d'éclairage (10) selon la revendication 6 ou 7,
**caractérisé en ce que** le dispositif de réglage comprend une caméra (16) qui est agencée pour détecter des gestes effectués par un utilisateur du dispositif d'éclairage, et comprend ou est relié à une unité de détermination qui est agencée pour associer les gestes à des commandes de fonctionnement prédéfinies, dans lequel le dispositif de réglage est agencé pour régler l'orientation d'une direction principale de rayonnement (30) respective des dispositifs lumineux (12) associés au dispositif de réglage sur la base des commandes de fonctionnement.

9. Dispositif d'éclairage (10) selon l'une des revendications 6 à 8,
**caractérisé en ce que** le dispositif de réglage comprend une interface de données ou est relié à celle-ci, laquelle est agencée pour recevoir des données d'opération concernant une opération à effectuer sur un patient,
dans lequel le dispositif de réglage est agencé pour régler une orientation d'une direction de rayonnement principale (30) respective des dispositifs lumineux (12) associés au dispositif de réglage sur la base des données d'opération.
